(19)

Europäisches
Patentamt
European
Patent Office
Office européen
des brevets

(11) **EP 3 815 544 A1**

(12) **EUROPEAN PATENT APPLICATION**
published in accordance with Art. 153(4) EPC

(43) Date of publication:
**05.05.2021 Bulletin 2021/18**

(21) Application number: **19825761.0**

(22) Date of filing: **27.06.2019**

(51) Int Cl.:
*A23L 33/125* (2016.01)   *A23L 2/52* (2006.01)
*A23L 33/145* (2016.01)   *A61K 31/715* (2006.01)
*A61K 36/06* (2006.01)   *A61P 1/04* (2006.01)
*A61P 1/10* (2006.01)   *A61P 3/00* (2006.01)
*A61P 3/02* (2006.01)   *A61P 3/04* (2006.01)
*A61P 5/30* (2006.01)   *A61P 15/00* (2006.01)
*A61P 17/00* (2006.01)   *A61P 19/02* (2006.01)
*A61P 21/00* (2006.01)   *A61P 25/18* (2006.01)
*A61P 25/22* (2006.01)   *A61P 25/24* (2006.01)
*A61P 25/26* (2006.01)   *A61P 29/02* (2006.01)
*A61P 43/00* (2006.01)

(86) International application number:
**PCT/JP2019/025659**

(87) International publication number:
**WO 2020/004568 (02.01.2020 Gazette 2020/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.06.2018   JP 2018123056**

(71) Applicant: **Asahi Group Holdings, Ltd.
Tokyo 130-8602 (JP)**

(72) Inventors:
• **TANIHIRO, Reiko
Moriya-shi, Ibaraki 302-0106 (JP)**
• **KAWAGUCHI, Kyosuke
Moriya-shi, Ibaraki 302-0106 (JP)**
• **MIZUNO, Megumi
Moriya-shi, Ibaraki 302-0106 (JP)**
• **OBA, Shunsuke
Moriya-shi, Ibaraki 302-0106 (JP)**

(74) Representative: **Hoffmann Eitle
Patent- und Rechtsanwälte PartmbB
Arabellastraße 30
81925 München (DE)**

(54) **EQUOL PRODUCTION PROMOTOR AND FOOD OR BEVERAGE COMPOSITION COMPRISING SAME FOR PROMOTION OF EQUOL PRODUCTION**

(57)    The present invention provides novel means for promoting equol production in the human intestine, or related novel means for improving premenstrual syndromes or improving skin condition changes associated with aging or skin problems. An equol production promoting agent of the present invention contains yeast mannan as an active ingredient. A premenstrual syndrome improving agent and a skin condition improving agent of the present invention contain the equol production promoting agent (yeast mannan). A food composition and a beverage composition for promoting equol production, improving premenstrual syndromes or improving skin conditions according to the present invention each contain the equol production promoting agent, the premenstrual syndrome improving agent or the skin condition improving agent.

**Description**

Technical Field

The present invention relates to an agent for promoting equol production in the intestine, and a food or beverage composition containing the agent as an active ingredient. The present invention also relates to uses of yeast mannan extracted from cell walls of yeast.

Background Art

Equol is a physiologically active substance which is a metabolic product of daidzein, a type of soybean isoflavone, and exhibits higher female hormone-like activity (i.e. estrogen activity) than soybean isoflavone itself. Equol has been heretofore reported to have effects of alleviation of menopause symptoms, improvement of skin conditions, and alleviation of discomforts caused by premenstrual syndromes (PMSs) and the like (e.g. Non-Patent Literature 1).

While humans do not have an enzyme which converts an equol precursor such as daidzein into equol, human intestinal bacteria (e.g. Bacteroides ovatus, Streptococcus intermedius and Streptococcus constellatus) have such an enzyme and produce equol in the intestine.

For increasing the concentration of equol in the human body, equol may be orally ingested directly, but equol is a compound not contained in general foods, there is little history of eating foods containing equol (therefore a form such as a supplement containing equol is needed), and orally ingested equol cannot be accumulated. On the other hand, as materials (compositions) for promoting equol production by intestinal bacteria, for example, those disclosed in Patent Literatures 1 to 3 are known. Specifically, Patent Literature 1 discloses an equol production promoting composition comprising aldonic acid or a derivative thereof as an active ingredient. Patent Literature 2 discloses a blood equol level elevating agent containing difructose anhydride and isoflavone. Patent Literature 3 discloses a composition for promoting equol production in the blood of an equol non-producer, the composition containing a nonfat milk solid and containing a daidzein-based isoflavone selected from daidzein, daidzin, malonyldaidzin, acetyldaidzin and succinyldaidzin, as such or in the form of a soybean embryonic axis extract or a purified product thereof (except for compositions as food, and compositions containing formononetin or microorganisms capable of producing equol by consuming daidzein).

However, any of the above-mentioned documents etc. does not either indicate or suggest that α-mannan or an α-mannan-protein complex extracted from yeast (collectively referred to as "yeast mannan" in the present description) contributes to promotion of equol production.

Citation List

Patent Literature

Patent Literature 1: WO 2007/148631
Patent Literature 2: JP 2006-143623 A (JP 4723849 B)
Patent Literature 3: WO 2008/146835 (JP 5617613 B)

Non Patent Literature

Non Patent Literature 1: Kenneth D. R. Setchelland Carlo Clerici, Equol: History, Chemistry, and Formation, The Journal of Nutrition, pp. 1355S-1362S, 2010 (doi: 10.3945/in. 109.119776)

Summary of Invention

Technical problem

An object of the present invention is to provide novel means for promoting equol production in the human intestine, or related novel means for improving premenstrual syndromes or improving skin condition changes associated with aging or skin problems. In another aspect, an object of the present invention is to provide novel uses of yeast mannan.

Solution to Problem

The present inventors have found that ingestion of yeast mannan can increase the amount of equol produced in the human intestine, furthermore, improve premenstrual syndromes, and improve skin condition changes associated with aging or skin problems. Yeast mannan is soluble in water, and forms an aqueous solution having a very low viscosity. Therefore, yeast mannan is a material suitable for use in mixture with a beverage or a food (hereinafter, collectively referred to as a "food/beverage").

That is, the present invention includes the following items.

[Item 1]

An equol production promoting agent comprising yeast mannan as an active ingredient.

[Item 2]

A food composition or a beverage composition for promoting equol production, comprising the equol production promoting agent described in item 1.

[Item 3]

A premenstrual syndrome improving agent comprising the equol production promoting agent described in item 1.

[Item 4]

A food composition or a beverage composition for improving premenstrual syndromes, comprising the premenstrual syndrome improving agent described in item 3.

[Item 5]

A skin condition improving agent comprising the equol production promoting agent described in item 1.

[Item 6]

A food composition or a beverage composition for improving skin conditions, comprising the skin condition improving agent described in item 5.

[Item 7]

Yeast mannan used for promoting equol production.

[Item 8]

Use of yeast mannan for producing a food composition or a beverage composition for promoting equol production.

[Item 9]

Yeast mannan used for improving premenstrual syndromes.

[Item 10]

Use of yeast mannan for producing a food composition or a beverage composition for improving premenstrual syndromes.

[Item 11]

Yeast mannan used for improving skin conditions.

[Item 12]

Use of yeast mannan for producing a food composition or a beverage composition for improving skin conditions.

The "equol production promoting agent" and the "food composition or beverage composition for promoting equol production" described above can be converted to, for example, "yeast mannan used (as an active ingredient (agent)) for promoting equol production", "use (application) of yeast mannan (as an active ingredient (agent)) for promoting equol production", "use of yeast mannan (as an active ingredient (agent)) for producing a food composition or a beverage composition for promoting equol production", or "a method for promoting equol production, comprising ingesting an effective amount of yeast mannan (contained in a food composition or a beverage composition) or causing the yeast mannan to be ingested (introducing the yeast mannan or causing the yeast mannan to be introduced into the intestine)", and matters related to the "equol production promoting agent" and the "food composition or beverage composition for promoting equol production" as described in the present description can be similarly applied. The "premenstrual syndrome improving agent" and the "food composition or beverage composition for improving premenstrual syndromes" described above can be converted to, for example, "yeast mannan used (as an active ingredient (agent)) for improving premenstrual syndromes", "use (application) of yeast mannan (as an active ingredient (agent)) for improving premenstrual syndromes", "use of yeast mannan (as an active ingredient (agent)) for producing a food composition or a beverage composition for improving premenstrual syndromes", or "a method for improving premenstrual syndromes, comprising ingesting an effective amount of yeast mannan (contained in a food composition or a beverage composition) or causing the yeast mannan to be ingested (introducing the yeast mannan or causing the yeast mannan to be introduced into the intestine)", and matters related to the "premenstrual syndrome improving agent" and the "food composition or beverage composition for improving premenstrual syndromes" as described in the present description can be similarly applied. The "skin condition improving agent" and the "food composition or beverage composition for improving skin conditions" described above can be converted to, for example, "yeast mannan used (as an active ingredient (agent)) for improving skin conditions", "use (application) of yeast mannan (as an active ingredient (agent)) for improving skin conditions", "use of yeast mannan (as an active ingredient (agent)) for producing a food composition or a beverage composition for skin conditions", or "a method for improving skin conditions, comprising ingesting an effective amount of yeast mannan (contained in a food composition or a beverage composition) or causing the yeast mannan to be ingested (introducing the yeast mannan or causing the yeast mannan to be introduced into the intestine)", and matters related to the "skin condition improving agent" and the "food composition or beverage composition for improving skin conditions" as described in the present description can be similarly applied.

In the present description, the equol production promoting agent, the premenstrual syndrome improving agent and the skin condition improving agent may be collectively referred to as a "agent of the present invention", or may be each referred to as "each agent of the present invention". The food composition and the beverage composition may be collectively referred to as a "food/beverage composition".

Advantageous Effects of Invention

Use of an equol production promoting agent containing yeast mannan as an active ingredient according to the present invention enables production of a food/beverage composition for promoting equol production, which is excellent in effect of promoting equol production in the intestine and which is easy to ingest. Since promoting equol production enables improvement of various symptoms of premenstrual syndromes and improvement of skin condition changes associated with aging or skin problems, the equol production promoting agent of the present invention and a food/beverage composition containing the equol production promoting agent can be applied to a premenstrual syndrome improving agent and a food/beverage composition containing the premenstrual syndrome improving agent, and a skin condition improving agent and a food/beverage composition containing the skin condition improving agent. While the amount of equol production (i.e. amount of metabolism from daidzein to equol) in the intestine varies between individuals as described in Patent Literatures 1 and 3, the equol production promoting agent etc. of the present invention can provide the effect of improving premenstrual syndromes or improving skin conditions by increasing the amount of equol production as compared to that before ingestion for a wide range of people.

Description of Embodiments

[Equol Production Promoting agent, Premenstrual Syndrome Improving Agent and Skin Condition Improving Agent]

The equol production promoting agent of the present invention contains yeast mannan, and may be one that contains only yeast mannan (i.e. consists of yeast mannan) as an active ingredient for the effect of promoting equol production in the intestine, or one that contains, in addition to yeast mannan, another active ingredient having an effect of promoting equol production as well.

The premenstrual syndrome improving agent of the present invention contains the above-described equol production promoting agent of the present invention (yeast mannan as an active ingredient for promoting equol production), and may be one that contains only yeast mannan (i.e. consists of yeast mannan) as an active ingredient for improving various symptoms of premenstrual syndromes (e.g. at least one of a total of 17 items in questions 1 and 2 as described in Examples below), or one that contains, in addition to yeast mannan, another active ingredient having an effect of improving premenstrual syndromes as well. The premenstrual syndrome (PMS) is a physical or psychological symptom beginning from a time of the premenstrual luteal phase, and is said to occur in 80% of females. Of PMS symptoms, conditions involving severer psychological symptoms and disturbing daily life are each called a premenstrual dysphoric disorder (PMDD), and proposed diagnostic criteria for these symptoms are described in "DSM-IV-TR" (Diagnostic and Statistical Manual of Mental Disorders) which are guidance of diagnostic criteria for psychological diseases in American Psychiatric Association.

The skin condition improving agent of the present invention contains the above-described equol production promoting agent of the present invention (yeast mannan as an active ingredient for promoting equol production), and may be one that contains only yeast mannan (i.e. consists of yeast mannan) as an active ingredient for improving skin conditions, or one that contains, in addition to yeast mannan, another active ingredient having an effect of improving skin conditions as well. Here, improvement of skin conditions includes improvement of skin condition problems, and improvement of skin condition changes associated with aging. Examples of the skin condition problems include acne, pimples, dryness, stickiness, roughness, redness, itchiness and various other symptoms from skin deterioration. The skin condition changes associated with aging means presenting with skin conditions different from the past although there is no problem in light of a relevant age. Examples of the skin condition changes associated with aging include a decrease in moisture content of the skin, a loss of skin elasticity, disordered skin textures, color unevenness, sagging, spots, wrinkles and visible skin pores.

In typical embodiments of the present invention, ingestion of yeast mannan as an active ingredient of the equol production promoting agent enables promotion of equol production as well as one or both of improvement of symptoms of premenstrual syndromes and improvement of skin condition changes. Therefore, in an embodiment of the present invention, the equol production promoting agent, the premenstrual syndrome improving agent and the skin condition improving agent can be embodied by the same agent.

The effect of promoting equol production is also related to effects other than the above-described effect of improving premenstrual syndromes and effect of improving skin conditions. Examples of such effects include effects exhibited by increasing the blood equol concentration, such as effects of improvement of menopausal disorders, an increase in bone density (prevention and/or treatment of osteoporosis), prevention and/or treatment of breast cancer or prostate cancer, suppression of an increase in blood sugar, suppression of an increase in blood cholesterol, suppression of an increase in blood neutral fat, reduction of body fat and visceral fat areas, and improvement of arterial sclerosis. Therefore, the equol production promoting agent of the present invention can be used for other purposes (or effects) as described above.

(1) Yeast Mannan

In the present invention, yeast mannan refers to a substance containing at least one of a yeast-derived "α-mannan" and a yeast-derived "α-mannan-protein complex". Both of the α-mannan and the α-mannan-protein complex are main components constituting yeast cell walls.

The above-mentioned "α-mannan" is a polysaccharide formed through polymerization of D-mannose via an α1,6-bond, an α1,2-bond, or an a1,3-bond, and a side chain formed of one or two or more mannopyranose moieties may be bonding to the main chain via an a1,2-bond or an a1,3-bond. This α-mannan is water-soluble.

On the other hand, the above-mentioned "α-mannan-protein complex" is a water-soluble glycoprotein in which one or two or more α-mannan chains are bonding to a protein locally present in yeast cell walls via an N-glycoside bond or an O-glycoside bond. As with the case with the above "α-mannan", each of the "α-mannan chains" bonding in the glycoprotein is a polysaccharide chain formed through polymerization of D-mannose via an α1,6-bond, an α1,2-bond, or an α1,3-bond, and a side chain formed of one or two or more mannopyranose moieties may be bonding to the main chain via an α1,2-bond or an α1,3-bond. The protein in the "α-mannan-protein complex" contains a peptide having two or more amino acid residues.

Yeast mannan may be collected in any manner as long as being derived from yeast. For example, yeast mannan formed by damaging yeast cell walls with an enzyme, chemical treatment, or the like may be separated and collected (extracted), and yeast mannan discharged by a yeast into a medium may be collected.

Yeast mannan is readily available as a commercially available product. Specific examples thereof include mannan from Saccharomyces cerevisiae M7504 (Sigma-Aldrich Japan). Alternatively, yeast mannan can be prepared from a yeast by using any known method as described in the following.

(2) Method for Preparing Yeast Mannan

Any known method can be used for preparing yeast mannan. For example, yeast mannan can be obtained through a step of preparing cell walls from a yeast by extracting yeast extract from a yeast used in production of any of beer, sake, bread, and so on to afford residual yeast cell walls, and a step of preparing yeast mannan from the residual yeast cell walls. Yeast cell walls sold under the product name "Kobo Saibo Heki (Yeast Cell Walls in English)" by Asahi Group Foods, Ltd. and other similar commercially available products may be used as a raw material of yeast mannan.

An example of the step of preparing yeast mannan from yeast cell walls is a step including a step of obtaining a crude extract of yeast mannan from yeast cell walls (herein, called the "crude extraction step") and, as necessary, an additional subsequent step of purifying the crude extract of yeast mannan to obtain a purified product of yeast mannan (herein, called the "purification step"). In some cases, a step of preparing cell walls from a yeast is performed before the crude extraction step; however, such a step is unnecessary in the case that yeast cell walls produced in advance are used as a raw material. In the crude extraction step, for example, methods of hot water (including dilute alkalis) extraction (e.g., see JP 1989(S64)-3479 B and JP 1983(S58)-109423 A), autodigestion (e.g., see JP 1983(S58)-57153 B), and enzymatic digestion with a cell-wall-digesting enzyme (e.g., see JP 1983(S59)-40126 B) can be used. In the purification step, for example, protein removal treatment with hydrochloric acid or the like, alcohol precipitation treatment, protease treatment, and treatment with chromatography can be performed.

Herein, crude extracts of yeast mannan and purified products of yeast mannan are collectively called "yeast mannan preparations". The above-described commercially available yeast mannan products are also included in the category of yeast mannan preparations. Various contaminants present in yeast cell walls or generated through treatment for preparation of yeast mannan (e.g., glucan, protein, mannose) may be contained in addition to yeast-derived α-mannan and/or an α-mannan-protein complex in yeast mannan preparations. The yeast mannan content of a yeast mannan preparation is not limited to particular values, and may be any content such that the yeast mannan contains such a content of yeast α-mannan, described later, that the operative advantageous effects of the present invention are exerted when the yeast mannan preparation is used as yeast mannan. Which method is used in the crude extraction step, whether the purification step is performed, which treatment is used if the purification step is performed, and so on may be appropriately determined so that a yeast mannan preparation containing a desired content of yeast α-mannan can be obtained. Those skilled in the art could prepare yeast mannan containing such a content of yeast α-mannan that the operative advantageous effects of the present invention are exerted without problem in accordance with a common method and conditions. In other words, yeast mannan prepared by those skilled in the art in accordance with a common method and conditions contains such a content of yeast α-mannan that the operative advantageous effects of the present invention are exerted without problem.

The yeast to be used as a raw material in the above method for preparing yeast mannan is not limited to a particular yeast, and may be any yeast whose cell walls contain the above-described α-mannan and the above-described α-mannan-protein complex as constituent components. The yeast may be any yeast of Saccharomyces spp., Shizosaccharomyces spp., Pichia spp., Candida spp., Kluyveromyces spp., Williopsis spp., Debaryomyces spp., Galactomyces spp., Torulaspora spp., Rhodotorula spp., Yarrowia spp., Zygosaccharomyces spp., and so on, and is preferably Saccharomyces sp. Those skilled in the art could culture a yeast that does not cause any problem in using the yeast as a raw material for preparing yeast mannan that exerts the operative advantageous effects of the present invention in accordance with a common method and conditions. In other words, a yeast cultured by those skilled in the art in accordance with a common method and conditions can be used as a raw material for preparing yeast mannan that exerts the operative advantageous effects of the present invention without problem.

The above-described yeast to be used as a raw material of yeast mannan is preferably an edible yeast, and more preferably a yeast used as a bread yeast or a beer yeast. Specific examples thereof include Saccharomyces cerevisiae, Schizosaccharomyces pombe, Saccharomyces pastorianus, Saccharomyces paradoxus, Saccharomyces mikatae, Saccharomyces bayanus, Saccharomyces kudriavzevii, Kluyveromyces lactis, Candida utilis, Candida albicans, Candida tropicalis, Candida lypolitica, and Candida sake. The above-described yeast to be used as a raw material of yeast mannan is preferably Saccharomyces cerevisiae, Saccharomyces pastorianus, or Saccharomyces bayanus.

(3) Method for Calculating Content Ratio of Yeast α-Mannan

The yeast mannan preparation obtained with the above preparation method may contain a yeast-derived α-mannan-protein complex (i.e., a protein moiety bonding to an α-mannan chain). Herein, yeast-derived α-mannan and the α-mannan chain part of a yeast-derived α-mannan-protein complex are collectively referred to as "yeast α-mannan". The content ratio of yeast α-mannan in yeast mannan somewhat varies among methods for preparing yeast mannan, but is in a generally constant range, typically, 30 to 95%. The content ratio of yeast α-mannan is not limited to particular values, and may be any value such that the operative advantageous effects of the present invention are exerted in using a yeast mannan preparation as yeast mannan. Those skilled in the art could prepare yeast mannan with such a content ratio of yeast α-mannan that the operative advantageous effects of the present invention are exerted without problem in accordance with a common method and conditions. In other words, yeast mannan prepared by those skilled in the art in accordance with a common method and conditions has such a content ratio of yeast α-mannan that the operative advantageous effects of the present invention are exerted without problem.

The content ratio of yeast α-mannan in a yeast mannan preparation is calculated in the following manner. First, mannose in sample (A) of a yeast mannan preparation and mannose in sample (B) of a hydrolysis product of the yeast mannan preparation are quantified. Here, sample (B) is a specimen obtained by treating sample (A) with sulfuric acid to hydrolyze all the glycans in sample (A) into monosaccharides and then neutralizing the resultant. Mannose in each specimen can be quantified, for example, by using high-performance liquid chromatography (HPLC), and may be quantified by using a method performed in the general incorporated foundation Japan Food Research Laboratories. Subsequently, the amount of mannose in sample (A) is subtracted from the amount of mannose in sample (B), and the resulting value is multiplied by 0.9 to exclude the amount of water bonded through hydrolysis of yeast α-mannan, giving a calculated value of the content of yeast α-mannan in the yeast mannan preparation. Finally, the ratio of the content of yeast α-mannan to the weight of sample (A) is calculated, thus determining the ratio of yeast α-mannan in the yeast mannan preparation, that is, the content ratio of yeast α-mannan. For each agent of the present invention, it is not necessary to use a yeast mannan preparation in which the purity of yeast α-mannan is 100%, and a yeast mannan preparation with a purity in a general range as described above may be used.

The form of each of the equol production promoting agent, the premenstrual syndrome improving agent and the skin condition improving agent of the present invention is not particularly limited, and may be a powdered form, or a solution form (dispersion liquid form) including water or another solvent. Typically, each agent of the present invention is used in mixture with a food/beverage composition used for a purpose corresponding to the effect of each agent, and therefore it is preferable that each agent have a form suitable for production of such a food/beverage composition.

The effect of each agent of the present invention, i.e. the effect of promoting equol production, the effect of improving premenstrual syndromes and the effect of improving skin conditions can be each evaluated by a known method or in accordance with a known method. The effect of promoting equol production can be evaluated by, for example, measuring the equol concentration in the urine of a test subject by an immunochromatography method, or measuring the equol concentration in the urine or blood of a test subject by HPLC (high performance liquid chromatography) or the like, and determining whether the equol concentration is increased by ingesting the equol production promoting agent (yeast mannan that is an active ingredient of the promoting agent). The effect of improving premenstrual syndromes can be evaluated by, for example, asking a test subject a question about the seriousness of a symptom of the premenstrual syndrome, and determining whether a score corresponding to an answer to the question is improved by ingesting the premenstrual syndrome improving agent (yeast mannan that is an active ingredient of the agent). The effect of improving skin conditions can be evaluated by, for example, asking a test subject a question about the seriousness of skin problems or the degree of skin condition changes associated with aging, or measuring an index value of skin conditions (e.g. skin moisture content, transepidermal water loss, skin elasticity, sebum content, spots, wrinkles, skin textures, color unevenness, skin pores, brown spots, redness, porphyrin content, the number of rashes and collagen content), and determining whether a score corresponding to an answer to the question or a measured value of the index value is improved by ingesting the skin condition improving agent (yeast mannan that is an active ingredient of the agent). For any of the effects, a test is conducted after groups (including a control group such as placebo) and conditions are appropriately set, and statistical processing (examination of whether there is a significant difference) is performed to make a reasonable evaluation.

[Food/Beverage Composition]

The food/beverage composition (food composition and beverage composition) for promoting equol production according to the present invention contains the equol production promoting agent of the present invention, and another ingredient adapted to the respective forms of the food composition and the beverage composition. The food/beverage composition for improving premenstrual syndromes and the food/beverage composition for improving skin conditions according to the present invention similarly contain the premenstrual syndrome improving agent and the skin condition improving agent of the present invention, and another ingredient adapted to the respective forms of the food composition and the beverage composition. In other words, the food/beverage composition for promoting equol production, the food/beverage composition for improving premenstrual syndromes and the food/beverage composition for improving skin conditions according to the present invention are food/beverage compositions containing the equol production promoting agent, the premenstrual syndrome improving agent and the skin condition improving agent of the present invention as ingredients (additives) for imparting the effects of promoting equol production, improvement of various symptoms of premenstrual syndromes and improvement of skin conditions, respectively.

A natural material or a processed product thereof which contains yeast mannan as an active ingredient of the equol production promoting agent (and the premenstrual syndrome improving agent and the skin condition improving agent containing the promoting agent) in the form of yeast having cell walls, but is not produced as a composition, for example a fermented food or brewed beverage itself in which yeast remains, is not the food-beverage composition of the present invention.

The form of the food/beverage composition is not particularly limited, and can be similar in form to various known foods/beverages, with which each agent of the present invention can be mixed. Examples of the form of the food product composition include supplements (dietary supplements) in tablets, capsules, powders (granules), and other dosage forms; soft drinks (e.g., carbonated drinks, non-alcoholic drinks, juices, coffee drinks, tea drinks, mineral waters, sports drinks), milk drinks, soymilk drinks, fermented milks, lactic acid bacteria drinks, cocoas, alcoholic drinks (e.g., beer, low-malt beer, other brewed alcoholic drinks, liqueur, Japanese sake, amazake, wine, shochu), drinks prepared from instant powders, and other drinks; bread, cereal, confectionery (e.g., biscuits, cookies, chocolates), processed products (e.g., katsuobushi, shiokara, kusaya), salted vegetables (nukadzuke, kimchi, pickles), fermented food products (e.g., natto, cheese, yogurt), seasonings (e.g., soy sauce, miso, vinegar), and other food products.

The content of each agent of the present invention in the food/beverage composition or the content of yeast mannan as an active ingredient thereof is not particularly limited as long as the agent or the yeast mannan has a content of yeast $\alpha$-mannan which allows the effect of each agent of the present invention, i.e. the effect of promoting equol production, the effect of improving premenstrual syndromes or effect of improving skin conditions, to be exhibited. In addition, the content of yeast $\alpha$-mannan in the food/beverage composition is not particularly limited as long as the effect of each agent of the present invention, i.e. the effect of promoting equol production, the effect of improving premenstrual syndromes or the effect of improving skin conditions, is exhibited. The contents of yeast mannan and yeast $\alpha$-mannan can be appropriately set in consideration of a form of the food/beverage composition, a method for producing the composition, and the like, and in consideration of the amount of an active ingredient per meal or per day, and the like if necessary. When a yeast mannan preparation is used as each agent of the present invention, by the weight of the yeast mannan preparation and the ratio of yeast $\alpha$-mannan contained in the yeast mannan preparation (content ratio of yeast $\alpha$-mannan), the content of the yeast mannan as an active ingredient can be adjusted to fall within a range which allows the effect of each agent of the present invention to be exhibited.

The raw materials or ingredients other than each agent of the present invention in a food/beverage composition of the present invention can be the same as in a conventional food/beverage composition.

The food/beverage composition of the present invention is not required to have soybean isoflavone (daidzein, or a glycoside or analog thereof such as daidzin, malonyldaidzin, acetyldaidzin or succinyldaidzin), which is a precursor of equol, as an essential ingredient, but if desired, a purified product of soybean isoflavone or a substance containing soybean isoflavone (e.g. a soybean-derived food/beverage such as soymilk) may be present as an ingredient.

The food/beverage composition of the present invention, which takes the form of a supplement, may contain, in addition to each agent of the present invention, various ingredients contained in a common or known supplement, for example at least one selected from an excipient, a sweetener, a colorant, a flavor and other ingredients. The content of each agent of the present invention in the supplement is not particularly limited and can be appropriately set as in a common supplement. For example, the content of the agent can be set so that the content of the yeast mannan preparation in the supplement is 5% or more or 10% or more and 75% or less based on the total mass of the supplement.

The content of the yeast mannan preparation, i.e. the equol production promoting agent, the premenstrual syndrome improving agent or the skin condition improving agent of the present invention, in the supplement may be 100%. The "supplement" in such an embodiment is a substance containing only each agent of the present invention, i.e. a substance consisting of only each agent of the present invention, and therefore is not the "food/beverage composition", but is one of the embodiments of the "equol production promoting agent, premenstrual syndrome improving agent or skin condition improving agent of the present invention", and can be considered to be used on the assumption of being ingested directly rather than being mixed with the food/beverage composition.

The food/beverage composition of the present invention, which take a beverage composition, may contain, in addition to each agent of the present invention, various ingredients contained in a common or known beverage, for example at least one selected from water (or carbonated water), coffee extract, tea leaf extract, fruit juice, other plant material extracts, milk, soymilk, fermented milk, a sweetener, an acidulant, a colorant and a flavor. The content of each agent of the present invention in the beverage composition is not particularly limited and can be appropriately set with consideration given to turbidity as in the case of a common beverage composition, if necessary. For example, the content of the agent can be set so that the content of yeast mannan in the beverage composition is 0.04% or more or 0.1% or more and 7.5% or less or 4% or less based on the total mass of the beverage composition.

Such a food/beverage composition of the present invention can be produced by the same method as in the case of a conventional food/beverage composition except that a step of adding each agent of the present invention as an ingredient is further carried out.

The food/beverage composition may be produced, for example, as a food with health claims (i.e., a food with nutrient function claims, a food for specified health uses, or a food with functional claims), or a therapeutic diet (i.e., a diet for treatment, or a diet prepared on the basis of a menu made by a nutritionist or the like with reference to a dietary slip from a physician), a diet for dietary therapy, or a care diet.

The approximate ingestion dose of the food/beverage composition per meal or per day can be appropriately set according to an age, a body weight, a sex or an index value before ingestion and the like of a subject expected to enjoy the effect of each agent of the present invention, or on the basis of nonclinical or clinical test results, and the like. The ingestion period of the food/beverage composition can be appropriately set, and it is preferable that the food/beverage composition be repeatedly and routinely ingested over a long period. In an aspect of the present invention, the food/beverage composition of the present invention is ingested in such a manner that the ingestion dose of yeast mannan as an active ingredient (also referred to as an "effective amount of yeast mannan") contained in the food/beverage composition is preferably 0.1 g/day or more, more preferably 0.2 g/day or more, still more preferably 0.3 g/day or more, and preferably 4 g/day or less, more preferably 2.5 g/day or less, still more preferably 1.5 g/day or less.

It is possible to directly or indirectly give an indication of an application of the food/beverage composition of the present invention, i.e. an indication that the food/beverage composition of the present invention is used for promoting equol production, improvement of premenstrual syndromes or improvement of skin conditions. As an example of the direct indication, a use of the food/beverage composition is described on tangible entities such as products themselves, packages, containers, labels and tags. As an example of the indirect indication, a use of the food/beverage composition is indicated through advertising campaigns with the aid of spots and a means such as websites, storefronts, exhibitions, signs, bulletin boards, newspapers, magazines, televisions, radios, males and e-mails. When the food/beverage composition of the present invention is produced as food with function claims, functions based on the effects (skin-moisturizing effect) of yeast mannan contained as a functional ingredient can be indicated by, for example, the following message: "This product contains yeast mannan. The yeast mannan is reported to have a function of helping maintain skin's moisture, and is recommendable for anyone who has a dry skin".

[Other Aspects of Invention]

In an aspect, the present invention provides a method for promoting production of equol in humans and mammals other than humans (e.g. laboratory animals such as mice, or pets), the method comprising ingesting an effective amount of yeast mannan or causing the yeast mannan to be ingested, in other words, introducing the yeast mannan or causing the yeast mannan to be introduced into the intestine. In an aspect, the present invention provides a method for improving the skins of humans and mammals other than humans (e.g. laboratory animals such as mice, or pets), the method comprising causing an effective amount of yeast mannan (or an equol production promoting agent containing the yeast mannan) to be ingested. In an aspect, the present invention provides a method for improving premenstrual syndromes of humans (particular females), the method comprising causing an effective amount of yeast mannan (or an equol production promoting agent containing the yeast mannan) to be ingested.

A person skilled in the art can convert the inventions of the "method for promoting equol production", the "method for improving skin conditions" and the "method for improving premenstrual syndromes" from the foregoing inventions of each agent of the present invention (equol production promoting agent, skin condition improving agent and premenstrual syndrome improving agent) and/or each food/beverage composition of the present invention (respective food compositions and beverage compositions for promoting equol production, for improving skin conditions and for improving premenstrual syndromes), and can similarly apply matters about each agent and/or each food/beverage composition described herein.

Examples

[Preparation of Yeast Mannan Preparation X]

(Yeast mannan preparation X: derived from Saccharomyces pastorianus)

10 kg of commercially available cell walls of yeast (trade name "Yeast Cell Wall" from Asahi Group Foods, Ltd.) were suspended in distilled water, and the suspension was adjusted to a pH of 11.0 by adding sodium hydroxide, and the mixture was then heated at 90°C for 12 hours. The supernatant was recovered through centrifugation, and adjusted to a pH of 5.5 by adding hydrochloric acid. The mixture was subjected to freeze-drying operation to give 2 kg of a yeast mannan preparation X.

(Content ratio of yeast α-mannan)

The amounts of mannose in the specimen (A) of the yeast mannan preparation X obtained above and mannose in the specimen (B) obtained by hydrolyzing the yeast mannan preparation X obtained above were each determined through commission to Japan Food Research Laboratories. The results showed that the content of mannose in the specimen (A) was a measurable limit value, and the content of mannose in the specimen (B) was 69 g/100 g of specimen. On the basis of these results, the content ratio of yeast α-mannan in the obtained yeast mannan preparation X was calculated by the above-described calculation method, and the result showed that the content ratio of the yeast α-mannan was 62%.

The specific procedure of the method for determining the amounts of mannose in the specimen (A) and the specimen (B) in Japan Food Research Laboratories is as follows. 0.6 g of each of the specimen (A) and the specimen (B) was collected and added to sulfuric acid with a concentration of 72%, and the mixture was stirred at room temperature for 1 hour. The specimen solution was diluted to have a sulfuric acid concentration of 4%, and then heated in an autoclave (121°C) for 1 hour. The specimen solution was cooled to room temperature, then neutralized, and quantitatively adjusted to 200 mL with water. The quantitatively adjusted specimen solution was filtered through a piece of filter paper, the filtrate was diluted with water by 50 times, and the diluted solution was further filtered through a membrane filter. This filtrate was analyzed by a high performance liquid chromatography method under the following operation conditions, and the concentration of mannose in the filtrate was measured to determine the amounts of mannose in the specimen (A) and the specimen (B).

Type of equipment: LC-20AD (Shimadzu Corporation)
Detector: Fluorescence Spectrometer RF-20Axs (Shimadzu Corporation)
Column: TSKgel SUGAR AXI, $\phi$4.6 mm $\times$ 150 mm (TOSOH CORPORATION)
Column temperature: 60°C
Mobile phase: 0.5 mol/L borate buffer solution (pH 8.7)
Flow rate: 0.4 mL/min
Injection amount: 20 $\mu$L
Fluorescence excitation wavelength: 320 nm
Fluorescence measurement wavelength: 430 nm
Post-column:

reaction solution: 1 w/v% L-arginine solution
reaction solution flow rate: 0.7 mL/min
reaction temperature: 150°C

[Examples 1 to 4 and Comparative Examples 1 and 2]

33 males and females in their twenties to forties were divided into three groups. Each group ingested a barley tea beverage containing 1 g or 2 g of the yeast mannan preparation X or a barley tea beverage containing a placebo (1 g of maltodextrin) (hereinafter, each barley tea beverage is also referred to as a "test beverage") once a day for four weeks, and before and after the ingestion, [A] a urine equol concentration was measured and [B] a skin problem score was recorded (Examples 1 and 2 and Comparative Example 1). In addition, 18 females in their twenties to forties were divided into three groups. As with [A] and [B] above, each group ingested a barley tea beverage containing 1 g or 2 g of the yeast mannan preparation X or a barley tea beverage containing a placebo (1 g of maltodextrin) once a day for four weeks, and [C] a premenstrual indefinite complaint score was recorded before and after the ingestion (Examples 3 and 4 and Comparative Example 2).

In evaluation of the above-described items involving equol producibility, all the test subjects are required to have ingested a sufficient amount of soybean isoflavone which is a precursor of equol. Thus, for urine collection both before and after ingestion of yeast mannan preparation, each test subject ingested 200 ml of a plain soymilk beverage containing 94 mg of isoflavone once a day for 3 days before urine collection in order to ingest soybean isoflavone which is a precursor of equol. The urine of each test subject was stored in a frozen state immediately after collection.

[A] Evaluation of Effect of Promoting Equol Production

The effect of promoting equol production was evaluated by the Wilcoxon signed rank test of the urine equol concentration of each test subject before and after ingestion of the yeast mannan preparation X. The urine equol concentration was measured by an immunochromatography method using urine thawed at room temperature. Table 1 shows the results of measuring the urine equol concentration and Wilcoxon signed rank test results. In the 2 g-yeast mannan preparation X ingestion group, the urine equol concentration significantly increased after ingestion of the test beverage. In the 1 g-yeast mannan preparation X ingestion group, the urine equol concentration tended to increase after ingestion of the test beverage. On the other hand, in the placebo ingestion group, there was no significant change in urine equol concentration between before and after ingestion of the test beverage.

[Table 1]

| | | Urine equol concentration [μM] (mean ± standard deviation) | | |
| | | Before ingestion | After ingestion | significant difference † (before and after ingestion) |
|---|---|---|---|---|
| Example 1 | Yeast mannan preparation X (yeast α-mannan content ratio = 1 g 62%) | 14.8±8.4 | 23.2±13.1 | p=0.075 |
| Example 2 | Yeast mannan preparation X (yeast α-mannan content ratio = 2 g 62%) | 15.8±8.1 | 28.7±12.9 | p=0.026 |
| Comparative Example 1 | Maltodextrin 1 g | 11.0±5.6 | 19.3±8.6 | |

†: Wilcoxon signed rank test

[B] Evaluation of Effect of Improving Skin Conditions

The effect of improving skin conditions was evaluated by the Wilcoxon signed rank test of four-case method-based answer scores (none = score 1, slight = score 2, moderate = score 3 and intense = 4) with respect to the following questions.
Question: Have you had a skin problem within recall of the condition in recent one month?
Table 2 shows the skin problem-related scores and Wilcoxon signed rank test results. In the 1 g- and 2 g-yeast mannan preparation X ingestion groups, the skin problem scores were significantly improved after ingestion of the test beverage. On the other hand, in the placebo ingestion group, there was no significant change in skin problem scores between before and after ingestion of the test beverage.

[Table 2]

| | | Skin problem score † (mean ± standard deviation) | | |
| | | Before ingestion | After ingestion | significant difference ‡ (before and after ingestion) |
|---|---|---|---|---|
| Example 1 | Yeast mannan preparation X (yeast α-mannan content ratio = 1 g 62%) | 1.82±0.23 | 1.27±0.14 | p=0.043 |
| Example 2 | Yeast mannan preparation X (yeast α-mannan content ratio = 2 g 62%) | 1.91±0.25 | 1.18±0.12 | p=0.018 |
| Comparative Example 1 | Maltodextrin 1 g | 1.55±0.16 | 1.37±0.20 | |

†: none = 1, slight = 2, moderate = 3 and intense = 4
‡: Wilcoxon signed rank test

[C] Evaluation of Effect of Improving Premenstrual Syndromes

The effect of improving premenstrual syndromes was evaluated by the Wilcoxon signed rank test of four-case method-based answer scores (none = score 1, slight = score 2, moderate = score 3 and intense = 4) with respect to the following 17 items. These questions are questions about psychological symptoms, physical symptoms and deteriorated social functions (hereinafter, also referred to as "premenstrual indefinite complaints"), and were prepared in accordance with a questionnaire (Yoshiko Miyaoka, Yoshie Akimoto, Kayoko Ueda and Toshiko Kamo: The reliability and validity of the newly developed PMDD scale: J Jp Soc Psychosom ObsLet Gynecol) Vol. 14, No. 2, pp. 194-210. October, 2009) prepared on the basis of the Japanese translation (Saburo Takahashi, Hiroshi Ono and Toshiyuki Someya (translation): Diagnostic and Statistical manual of mental disorders, Revision, Tokyo: IGAKU-SHOIN, 2003) of proposed diagnostic criteria for premenstrual dysphoric disorder (PMDD) of DSM-IV-TR which are guidance of diagnosis criteria for mental diseases in American Psychiatric Association (American Psychiatric Association: Diagnostic and Statistical Manual of Mental Disorders, Fourth Edition Text Revision: DSM-IV-TR. Washington DC: American Psychiatric Association, 2000).

Question 1: Have you had, in the recent menstrual cycle, the following symptoms beginning one or two weeks before the start of menstruation and disappearing two or three days after the start of menstruation?

(1) Feeling depressed or feeling hopeless.
(2) Feeling anxious or feeling nervous.
(3) Easily moved to tears or suddenly feeling sad.
(4) Easily moved to anger, feeling annoyed, or taking things out on others.
(5) Loosing interest (in work, school, hobbies and the like).
(6) Subjective sense of difficulty in concentration.
(7) Tired easily or lacking spirit.
(8) Having an increased appetite or needing a specific kind of food (e.g. sweets).
(9) Sleeping much more than usual, or feeling very sleepy.
(10) Feeling more difficult to sleep than usual.
(11) Feeling unable to control yourself.
(12) Having any of the following physical symptoms: breast tenderness or swelling, a sensation of "bloating", abdominal pain, headache, joint pain, muscle pain, sense of having a swelling body, body weight gain and constipation.
Question 2: Please answer the question below if you have had at least one of the symptoms listed above (slight to intense). Did you have the following daily activities been hindered during the duration of the symptoms?
(13) Work efficiency has been hindered (workplace or school).
(14) Housework has been hindered.
(15) The relationship with persons on the workplace has been hindered (examples of how the relationship is hindered: avoiding associations; taking things out on others; and quarreling).
(16) The relationship with family members has been hindered.
(17) The relationship with friends and acquaintances has been hindered.

Table 3 shows PMDD summary scores obtained by summing scores for the total of 17 items related to premenstrual indefinite complaints for each test subject, and Wilcoxon signed rank test results. In the 1 g- and 2 g-yeast mannan preparation X ingestion groups, the scores were significantly improved after ingestion of the test beverage.

[Table 3]

| | | PMDD summary score † (mean ± standard deviation) | | |
| --- | --- | --- | --- | --- |
| | | Before ingestion | After ingestion | significant difference ‡ (before and after ingestion) |
| Example 3 | Yeast mannan preparation X (yeast α-mannan content ratio = 1 g 62%) | 28.2±3.0 | 23.5±2.8 | p=0.043 |
| Example 4 | Yeast mannan preparation X (yeast α-mannan content ratio = 2 g 62%) | 23.8±1.4 | 18.2±0.5 | p=0.028 |
| Comparative Example 2 | Maltodextrin 1 g | 22.8±1.8 | 22.0±1.8 | |

†: Sum of scores for a total of 17 items related to premenstrual problems (none = 1, slight = 2, moderate = 3 and intense = 4)
‡: Wilcoxon signed rank test

Tables 4, 5, 6 and 7 show the scores and Wilcoxon signed rank test results for items (4), (8), (9) and (12), respectively, among individual scores for the total of 17 items. For the item (12) (physical symptoms (Table 7)), the scores were significantly improved after ingestion of the test beverage in the 2 g-yeast mannan preparation X ingestion group. For the three items (4) (annoyed (Table 4)), (8) (appetite (Table 5)) and (9) (sleepy (Table 6)), the scores tended to be improved after ingestion of the test beverage in the 1 g-yeast mannan preparation X ingestion group.

[Table 4]

| | | Score for item (4) † (mean ± standard deviation) | | |
| --- | --- | --- | --- | --- |
| | | Before ingestion | After ingestion | significant difference ‡ (before and after ingestion) |
| Example 3 | Yeast mannan preparation X (yeast α-mannan content ratio = 1 g 62%) | 2.17±0.40 | 1.33±0.21 | p=0.068 |
| Example 4 | Yeast mannan preparation X (yeast α-mannan content ratio = 2 g 62%) | 1.33±0.21 | 1.00±0.00 | |
| Comparative Example 2 | Maltodextrin 1g | 1.33±0.21 | 1.33±0.21 | |

†: Easily moved to anger, feeling annoyed, or taking things out on others / none = 1, slight = 2, moderate = 3 and intense = 4
‡: Wilcoxon signed rank test

[Table 5]

| | | Score for item (8) † (mean ± standard deviation) | | |
| --- | --- | --- | --- | --- |
| | | Before ingestion | After ingestion | significant difference ‡ (before and after ingestion) |
| Example 3 | Yeast mannan preparation X (yeast α-mannan content ratio = 1 g 62%) | 2.17±0.48 | 1.33±0.21 | p=0.068 |
| Example 4 | Yeast mannan preparation X (yeast α-mannan content ratio = 2 g 62%) | 1.67±0.33 | 1.17±0.17 | |
| Comparative Example 2 | Maltodextrin 1 g | 1.50±0.22 | 1.33±0.21 | |

†: Having an increased appetite or needing a specific kind of food (e.g. sweets) / none = 1, slight = 2, moderate = 3 and intense = 4
‡: Wilcoxon signed rank test

[Table 6]

| | | Score for item (9) † (mean ± standard deviation) | | |
| --- | --- | --- | --- | --- |
| | | Before ingestion | After ingestion | significant difference ‡ (before and after ingestion) |
| Example 3 | Yeast mannan preparation X (yeast α-mannan content ratio = 1 g 62%) | 2.50±0.43 | 1.67±0.33 | p=0.068 |
| Example 4 | Yeast mannan preparation X (yeast α-mannan content ratio = 2 g 62%) | 1.83±0.17 | 1.50±0.34 | |
| Comparative Example 2 | Maltodextrin 1 g | 2.33±0.33 | 2.00±0.45 | |

†: Sleeping much more than usual, or feeling very sleepy / none = 1, slight = 2, moderate = 3 and intense = 4
‡: Wilcoxon signed rank test

[Table 7]

| | | Score for item (12) † (mean ± standard deviation) | | |
| | | Before ingestion | After ingestion | significant difference ‡ (before and after ingestion) |
| --- | --- | --- | --- | --- |
| Example 3 | Yeast mannan preparation X (yeast α-mannan content ratio = 1 g 62%) | 2.67±0.42 | 2.17±0.31 | |
| Example 4 | Yeast mannan preparation X (yeast α-mannan content ratio = 2 g 62%) | 3.17±0.31 | 1.33±0.21 | p=0.028 |
| Comparative Example 2 | Maltodextrin 1 g | 1.67±0.21 | 1.83±0.40 | |

†: Having any of the following physical symptoms: breast tenderness or swelling, a sensation of "bloating", abdominal pain, headache, joint pain, muscle pain, sense of having a swelling body, body weight gain and constipation/none = 1, slight = 2, moderate = 3 and intense = 4

‡: Wilcoxon signed rank test

[Preparation of Yeast Mannan Preparations A, B and C]

(Yeast mannan preparation A: derived from Saccharomyces pastorianus)

10 kg of commercially available cell walls of yeast (trade name "Yeast Cell Wall" from Asahi Group Foods, Ltd.) were suspended in distilled water, and the suspension was adjusted to a pH of 11.0 by adding sodium hydroxide, and the mixture was then heated at 90°C for 12 hours. The supernatant was recovered though centrifugation, and adjusted to a pH of 5.5 by adding hydrochloric acid. The mixture was subjected to freeze-drying operation to give 2 kg of a yeast mannan preparation A. The content ratio of yeast α-mannan in the obtained yeast mannan preparation A was calculated by the above-described calculation method, and the result showed that the content ratio of the yeast α-mannan was 50.5%.

(Yeast mannan preparation B: derived from Saccharomyces cerevisiae)

A commercially available reagent (trade name "Mannan from Saccharomyces cerevisiae M7504 (Sigma-Aldrich Japan) was defined as a yeast mannan preparation B. The content ratio of yeast α-mannan in the yeast mannan preparation B was calculated by the above-described calculation method, and the result showed that the content ratio of the yeast α-mannan was 78.8%.

(Yeast mannan preparation C: derived from Candida utilis)

Candida utilis (IF00626) was inoculated in 8.0 L of YPD culture medium, and the culture medium was dispensed into 2.0 L conical flasks in such a manner that the flasks each contained 0.50 L of the culture medium. Shaking culture was performed at 25°C and 120 rpm for 24 hours. The culture solutions were collected, suspended in distilled water, the suspension was heated at 100°C for 10 minutes, and the supernatant was then removed through centrifugation to prepare a cell wall fraction. The obtained cell wall fraction was suspended in distilled water, and the suspension was adjusted to a pH of 11.0 by adding sodium hydroxide, and then heated at 90°C for 12 hours. The supernatant was recovered through centrifugation, and adjusted to a pH of 5.5 by adding hydrochloric acid. The mixture was subjected to freeze-drying operation to give 3.7 g of a yeast mannan preparation C. The YPD culture medium used had the following composition, and the pH of the YPD culture medium was not adjusted.

Yeast extract: 10 g
Peptone: 10 g
Glucose: 20 g
Distilled water: 1 L

[Example 5 and Comparative Example 3]

25 females in their thirties to forties, who recognized their skin dryness, were divided into two groups. Each group ingested a tablet containing 1230 mg of the yeast mannan preparation A or a control tablet containing 1230 mg of maltose (hereinafter, the tablet and the control tablet are referred to as "test food" and "control food", respectively) once a day for eight weeks. Before and after ingestion of the test food and the control food, [D] a transepidermal water loss was measured and [E] an equol conversion efficient was calculated from the urine equol concentration (Example 5 and Comparative Example 3). The amount of yeast $\alpha$-mannan in the test food ingested a day is 0.62 g.

[D] Measurement of Transepidermal Water Loss

The transepidermal water loss, which is the amount of water evaporated from the body through the horny cell layer without being recognized, is widely used as an index of a skin-moisturizing level. A test subject was placed at rest for 30 minutes in a constant-temperature and constant-humidity room kept at a temperature of 20 to 22°C and a humidity of 50±5%, and the transepidermal water loss on the inner side of the forearm was then measured using Tewameter TM300MP (Courage + Khazaka (CK) electronic GmbH) with the test subject placed in lateral position. The skin-moisturizing effects of the test food and the control food were evaluated by t-tests on the transepidermal water losses after eight-week ingestion of the test food and the control food, the transepidermal water losses before ingestion of the test food and the control food and the amount of change in transepidermal water loss for each of the test food and the control food for the test food ingestion group (Example 5) and the control food ingestion group (Comparative Example 3). Table 8 shows the transepidermal water losses and the amount of change in the transepidermal water loss for each group.

[Table 8]

| | | Before ingestion | | After eight-week ingestion | | Value after eight-week ingestion - value before ingestion | |
|---|---|---|---|---|---|---|---|
| | Transepidermal water loss (g/h/m$^2$) | Mean | Standard deviation | Mean | Standard deviation | Mean | Standard deviation |
| Example 5 | Test food Yeast $\alpha$-mannan 0.62 g/day (n=12) | 6.36 | 0.20 | 5.46 | 0.34 | -0.90 | 0.33 |
| Comparative Example 3 | Control food Maltose (n=13) | 7.11 | 0.34 | 7.40 | 0.38 | 0.30 | 0.35 |
| | Significant difference between groups (unpaired t-test) | None (p=0.079) | | p<0.001 | | p=0.020 | |

While there was no statistically significant difference in the transepidermal water losses between the groups before ingestion of the test food and the control food, the test food ingestion group showed a statistically significantly lower value than the control food ingestion group after eight-week ingestion of the test food or the control food. For the amount of change in transepidermal water loss between before and after ingestion of the test food and the control food, the test food ingestion group showed a statistically significantly lower value than the control food ingestion group.

[E] Calculation of Equol Conversion Efficiency from Urine Equol Concentration

The concentrations of daidzein, dihydrodaidzein and equol in the urine were measured by an enzyme HPLC (high performance liquid chromatography) method as described later, and an equol conversion efficiency was calculated using the following calculation expression.

```
equol conversion efficiency (%) = {equol/(daidzein +
dihydrodaidzein + equol)} * 100
```

In evaluation of equol conversion efficiency, all the test subjects are required to have ingested a sufficient amount of soybean isoflavone which is a precursor of equol. Thus, for urine collection both before and after ingestion of the test food or the control food, each test subject ingested a tablet containing 25 mg of soybean isoflavone once a day for 3 days before urine collection in order to ingest soybean isoflavone which is a precursor of equol. The urine of each test subject was stored in a frozen state immediately after collection.

(Enzyme HPLC Method)

The amounts of daidzein, dihydrodaidzein and equol in the urine were determined in accordance with the enzyme HPLC method by Redruello, B. et al. (Redruello, B. et al., A novel UHPLC method for the rapid and simultaneous determination of daidzein, genistein and equol in human urine, Journal of Chromatography B (2015), Volume 1005 (15 November 2015), pages 1-8).

500 $\mu$l of 0.2 M sodium acetate (pH 5.2) and 4 $\mu$l of $\beta$-glucuronidase/arylsulfatase (Sigma-Aldrich Japan) were added to 500 $\mu$l of urine thawed at room temperature. The mixture was reacted at 37°C for 3 hours. After completion of the reaction, 4-hydroxybenzophenone as an internal standard substance was added to a final concentration of 10 $\mu$g/ml, and the mixture was extracted with 3 ml of ethyl acetate twice. The extract was solidified by drying under reduced pressure, and then dissolved in 500 $\mu$l of a DMSO/methanol solution (1 : 1), and the solution was appropriately diluted, and subjected to HPLC analysis. The HPLC was performed under the following conditions.

    Apparatus: ACQUITY UPLC (manufactured by Waters Corporation)
    Column: ACQUITY UPLC BEH C18, 1.7 $\mu$m and 2.1 $\times$ 100 mm Column (manufactured by Waters Corporation)
    Detection: Ultraviolet absorptiometer (measurement
    wavelength: 280 nm)
    Column temperature: 40°C
    Mobile phase:

        Liquid A: water + 5 mM ammonium formate
        Liquid B: methanol + 5 mM ammonium formate

    Flow rate: 0.4 mL/min
    Sample injection amount: 1 $\mu$l

As standard products, daidzein (manufactured by Carbosynth Ltd.), dihydrodaidzein (manufactured by Cayman Chemical Company), equol (manufactured by Tocris Bioscience) and 4-hydroxybenzophenone (Tokyo Chemical Industry Co., Ltd.) were used.

For the equol conversion efficiency after ingestion of the test food or the control food as calculated by the above-described method, the test food ingestion group showed a statistically significantly higher value than the control food ingestion group.

[Examples 6 and 7 and Comparative Examples 4 and 5]

Fresh feces from an ICR mouse were suspended in 30 times their volume of GAM culture medium, and the suspension was filtered through a filter with a pore size of 100 $\mu$m (manufactured by FALCON Corporation). 3 ml of the filtrate was inoculated in 7 ml of GAM culture medium and cultured in an anaerobic chamber at 37°C for 24 hours. After the cultivation, the cultured product was mixed with 1/2 times its volume of a 80% glycerol solution, and the mixture was stored in a frozen state at -80°C as a mouse excrement suspension.

The yeast mannan preparation A was added to GAM without dextrose culture medium in such a manner that the final concentration of yeast $\alpha$-mannan was 1.0%. The mouse excrement suspension was seeded in the culture medium containing the yeast mannan preparation A, and anaerobically cultured at 37°C for 24 hours to be activated. The activated culture solution was centrifuged to remove the culture supernatant. Daidzein (manufactured by Carbosynth Ltd.) was added to the GAM without dextrose culture medium in such a manner that the final concentration of the daidzein was 100 $\mu$M. The culture solution freed of the supernatant was resuspended in the culture medium containing the daidzein. Here, the yeast mannan preparation A (Examples 6a and 6b) or the yeast mannan preparation B (Examples 7a and 7b) was added in such a manner that the final concentration of the yeast $\alpha$-mannan was 1.0% or 0.5%. Each of the mixtures was anaerobically cultured at 37°C for 48 hours, and the equol concentration after the cultivation was measured by the above-described enzyme HPLC method. A blank section free of the yeast mannan preparation (Comparative Example 4) and a mannose-containing section containing mannose being a constituent monosaccharide of $\alpha$-mannan at 1.0% (trade name "D(+)-Mannose" from FUJIFILM Wako Pure Chemical Corporation) instead of the yeast mannan preparation (Comparative Example 5) were provided, and the equol concentrations of these sections were compared with the measured equol concentrations.

The HPLC was performed under the following conditions.

Apparatus: ACQUITY UPLC (manufactured by Waters Corporation)
Column: ACQUITY UPLC BEH C18, 1.7 μm and 2.1 × 100 mm Column (manufactured by Waters Corporation)
Detection: Ultraviolet absorptiometer (measurement
wavelength: 280 nm)
Column temperature: 40°C
Mobile phase:

Liquid A: water + 5 mM ammonium formate
Liquid B: methanol + 5 mM ammonium formate

Flow rate: 0.4 mL/min
Sample injection amount: 1 μl

Table 9 shows the results from Examples 6a and 6b, Examples 7a and 7b and Comparative Examples 4 and 5.

[Table 9]

| | | Equol concentration (μM) |
|---|---|---|
| Comparative Example 4 | Blank | 9 |
| Example 6a | 1.0% Yeast α-mannan (Yeast mannan preparation A) | 29 |
| Example 6b | 0.5% Yeast α-mannan (Yeast mannan preparation A) | 23 |
| Example 7a | 1.0% Yeast α-mannan (Yeast mannan preparation B) | 22 |
| Example 7b | 0.5% Yeast α-mannan (Yeast mannan preparation B) | 15 |
| Comparative Example 5 | 1.0% Mannose | 2 |

Results with n = 3

For both the yeast mannan preparations A and B, the values of the equol concentration after the cultivation were higher in the section containing the yeast mannan preparation A or B than in the blank section (Examples 6a and 6b, Examples 7a and 7b and Comparative Example 4). The value of the equol concentration after the cultivation was higher in the section containing yeast α-mannan at 1.0% than in the section containing yeast α-mannan at 0.5%, and it was confirmed that the effect was enhanced with an increase in concentration. On the other hand, the value of the equol concentration after the cultivation was not higher in the section containing mannose at 1.0% than in the blank section (Comparative Examples 4 and 5).

[Examples 8 and 9 and Comparative Examples 6 and 7]

Fresh feces from an ICR mouse were suspended in 30 times their volume of GAM culture medium, and the suspension was filtered through a filter with a pore size of 100 μm (manufactured by FALCON Corporation). 3 ml of the filtrate was inoculated in 7 ml of GAM culture medium and cultured in an anaerobic chamber at 37°C for 24 hours. After the cultivation, the cultured product was mixed with 1/2 times its volume of a 80% glycerol solution, and the mixture was stored in a frozen state at -80°C as a mouse excrement suspension.

The yeast mannan preparation A was added to GAM without dextrose culture medium in such a manner that the final concentration of yeast α-mannan was 1.0%. The mouse excrement suspension was seeded in the culture medium containing the yeast mannan preparation A, and anaerobically cultured at 37°C for 24 hours to be activated. The activated culture solution was centrifuged to remove the supernatant. Daidzein (manufactured by Carbosynth Ltd.) was added to the GAM without dextrose culture medium in such a manner that the final concentration of the daidzein was 100 μM. The culture solution freed of the supernatant was resuspended in the culture medium containing the daidzein. Here, the yeast mannan preparation A or C was added in such a manner that the final concentration of the yeast α-mannan was 1.0% (Examples 8 and 9). The mixture was anaerobically cultured at 37°C for 48 hours, and the equol concentration after the cultivation was measured by the above-described enzyme HPLC method. The measured equol concentrations were compared with the equol concentrations of a blank section free of the yeast mannan preparation (Comparative Example 6) and a mannose-containing section containing mannose being a constituent monosaccharide of α-mannan at 1.0% (trade name "D(+)-Mannose" from FUJIFILM Wako Pure Chemical Corporation) instead of the yeast mannan preparation (Comparative Example 7).

The HPLC was performed under the following conditions.

Apparatus: ACQUITY UPLC (manufactured by Waters Corporation)

Column: ACQUITY UPLC BEH C18, 1.7 μm and 2.1 × 100 mm Column (manufactured by Waters Corporation)

Detection: Ultraviolet absorptiometer (measurement

wavelength: 280 nm)

Column temperature: 40°C

Mobile phase:

Liquid A: water + 5 mM ammonium formate

Liquid B: methanol + 5 mM ammonium formate

Flow rate: 0.4 mL/min

Sample injection amount: 1 μl

Table 10 shows results from Examples 8 and 9 and Comparative Examples 6 and 7.

[Table 10]

|  |  | Equol concentration (μM) |
| --- | --- | --- |
| Comparative Example 6 | Blank | 7 |
| Example 8 | 1.0% Yeast α-mannan (Yeast mannan preparation A) | 13 |
| Example 9 | 1.0% Yeast α-mannan (Yeast mannan preparation C) | 17 |
| Comparative Example 7 | 1.0% Mannose | 5 |

Results with n = 3

The results showed that for both the yeast mannan preparations A and C, the values of the equol concentration after the cultivation were higher in the section containing the yeast mannan preparation A or C than in the blank section (Examples 8 and 9 and Comparative Example 6). On the other hand, the value of the equol concentration after the cultivation was not higher in the section containing mannose at 1.0% than in the blank section (Comparative Examples 6 and 7) .

**Claims**

1.    An equol production promoting agent comprising yeast mannan as an active ingredient.

2.    A food composition or a beverage composition for promoting equol production, comprising the equol production promoting agent according to claim 1.

3.    A premenstrual syndrome improving agent comprising the equol production promoting agent according to claim 1.

4.    A food composition or a beverage composition for improving premenstrual syndromes, comprising the premenstrual syndrome improving agent according to claim 3.

5.    A skin condition improving agent comprising the equol production promoting agent according to claim 1.

6.    A food composition or a beverage composition for improving skin conditions, comprising the skin condition improving agent according to claim 5.

7.    Yeast mannan used for promoting equol production.

8.    Use of yeast mannan for producing a food composition or a beverage composition for promoting equol production.

**9.** Yeast mannan used for improving premenstrual syndromes.

**10.** Use of yeast mannan for producing a food composition or a beverage composition for improving premenstrual syndromes.

**11.** Yeast mannan used for improving skin conditions.

**12.** Use of yeast mannan for producing a food composition or a beverage composition for improving skin conditions.

## INTERNATIONAL SEARCH REPORT

| International application No. |
|---|
| PCT/JP2019/025659 |

A. CLASSIFICATION OF SUBJECT MATTER
Int.Cl. see extra sheet

According to International Patent Classification (IPC) or to both national classification and IPC

B. FIELDS SEARCHED

Minimum documentation searched (classification system followed by classification symbols)
Int.Cl. A23L2/00-35/00, A61K6/00-51/12, A61K36/06, A61P1/00-43/00

Documentation searched other than minimum documentation to the extent that such documents are included in the fields searched
| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS
(STN)

C. DOCUMENTS CONSIDERED TO BE RELEVANT

| Category* | Citation of document, with indication, where appropriate, of the relevant passages | Relevant to claim No. |
|---|---|---|
| X | JP 63-119427 A (KAO CORP.) 24 May 1988, claim 1, page 2, lower right column, lines 1-4, page 6, upper left column, lines 3-5, page 6, upper left column, line 15 to upper right column, line 6 (Family: none) | 5-7, 9, 11-12 |
| X | JP 2001-55338 A (KIRIN BREWERY CO., LTD.) 27 February 2001, claims 8-9, adjustment example 4, test example 4, fig. 4 & US 2002/0155126 A1, claims 8-9, preparation 4, test example 4, fig. 4 | 5-6 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 September 2019 (12.09.2019) | 24 September 2019 (24.09.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

# EP 3 815 544 A1

**INTERNATIONAL SEARCH REPORT**

| International application No. |
|---|
| PCT/JP2019/025659 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2010-540421 A (LESAFFRE ET COMPAGNIE) 24 December 2010, claims 8-9, paragraph [0002], example 1 & US 2011/0034393 A1, claims 8-9, paragraph [0002], example 1 & WO 2009/074735 A2 & EP 2203153 A2 & KR 10-2010-0058614 A & CN 101815500 A | 5, 7, 9, 11 |
| X | JP 61-271209 A (POLA CHEMICAL INDUSTRIES, INC.) 01 December 1986, claims 1-2, page 3, upper right column, line 4 to page 5, upper right column, line 4 (Family: none) | 5, 7, 9, 11 |
| Y | JP 2015-500802 A (CJ CHEILJEDANG CORPORATION) 08 January 2015, claims 1-2, 5, paragraphs [0007], [0023], [0027], [0038] & US 2014/0329286 A1, claims 1-2, 5, paragraphs [0006], [0023], [0026], [0037] & WO 2013/081294 A1 & EP 2787071 A1 & KR 10-1307947 B1 | 1-12 |
| Y | CUSKIN, Fiona et al., "Human gut Bacteroidetes can utilize yeast mannan through a selfish mechanism", Nature, 2015, vol. 517, pp. 165-169 page 165, right column, lines 18-23, fig. 1 | 1-12 |
| Y | JP 2017-163980 A (TAKARA BIO INC.) 21 September 2017, paragraph [0030] (Family: none) | 5-6, 11-12 |
| A | JP 2008-541700 A (SENSIENT FLAVORS INC.) 27 November 2008, fig. 2 & US 2006/0263415 A1, fig. 2 & WO 2006/121803 A1 & EP 1877447 A1 & CN 101184780 A | 1-12 |
| A | TAMURA, Motoi et al., "Konjac Glucomannan Consumption May Enhance Equol Production in Mice", Food Sci. Technol. Res., 2005, vol. 11, pp. 376-379 | 1-12 |
| E, A | WO 2019/142844 A1 (ASAHI GROUP HOLDINGS, LTD.) 25 July 2019 (Family: none) | 1-12 |
| E, A | WO 2019/142846 A1 (ASAHI GROUP HOLDINGS, LTD.) 25 July 2019 (Family: none) | 1-12 |

Form PCT/ISA/210 (continuation of second sheet) (January 2015)

Form PCT/ISA/210 (extra sheet) (January 2015)

| **INTERNATIONAL SEARCH REPORT** | International application No. |
|---|---|
| | PCT/JP2019/025659 |

CLASSIFICATION OF SUBJECT MATTER
A23L33/125(2016.01)i,      A23L2/52(2006.01)i,      A23L33/145(2016.01)i,
A61K31/715(2006.01)i,      A61K36/06(2006.01)i,      A61P1/04(2006.01)i,
A61P1/10(2006.01)i,      A61P3/00(2006.01)i,      A61P3/02(2006.01)i,
A61P3/04(2006.01)i,      A61P5/30(2006.01)i,      A61P15/00(2006.01)i,
A61P17/00(2006.01)i,      A61P19/02(2006.01)i,      A61P21/00(2006.01)i,
A61P25/18(2006.01)i,      A61P25/22(2006.11)i,      A61P25/24(2006.01)i,
A61P25/26(2006.01)i, A61P29/02(2006.01)i, A61P43/00(2006.01)i

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007148631 A
- JP 2006143623 A
- JP 4723849 B
- WO 2008146835 A
- JP 5617613 B

- JP 1989S643479 B
- JP 1983S58109423 A
- JP 1983S5857153 B
- JP 1983S5940126 B

**Non-patent literature cited in the description**

- **YOSHIKO MIYAOKA ; YOSHIE AKIMOTO ; KAYOKO UEDA ; TOSHIKO KAMO.** The reliability and validity of the newly developed PMDD scale. *J Jp Soc Psychosom ObsLet Gynecol,* October 2009, vol. 14 (2), 194-210
- translation): Diagnostic and Statistical manual of mental disorders. **SABURO TAKAHASHI ; HIROSHI ONO ; TOSHIYUKI SOMEYA.** Revision. IGAKU-SHOIN, 2003

- Text Revision: DSM-IV-TR. American Psychiatric Association: Diagnostic and Statistical Manual of Mental Disorders. American Psychiatric Association, 2000
- **REDRUELLO, B. et al.** A novel UHPLC method for the rapid and simultaneous determination of daidzein, genistein and equol in human urine. *Journal of Chromatography B,* 15 November 2015, vol. 1005, 1-8